# EUROPEAN PATENT APPLICATION

(11) **EP 1 958 578 A1**
(43) Date of publication of application: **20.08.2008**
(21) Application number: 08002638.8
(22) Date of filing: 13.02.2008
(51) Int. Cl.: A61B 17/70

(54) **Connector**

(30) Priority: 14.02.2007 JP 2007033967
(71) Applicant: Showa Ika Kohgyo Co., Ltd., Toyohashi-shi Aichi 441-8026 (JP)
(72) Inventor: Laager, Charles-Marc, 20148 Milano (IT); Boriani, Stefano, 40121 Bologna (IT); Ali, Giuseppe, 40134 Bologna (IT); Oribe, Kazuya, Minato-ku Tokyo 105-0011 (JP)
(74) Representative: Behm, Sonja Marianne

(57) **Abstract**

A connector (1) for vertebra connection members (2) that connect a plurality of vertebrae via a rod (6) includes: a connector body (3) that has formed therein a rod orifice (14) into which the rod is inserted, a fixing member orifice (16) into which a fixing member (15) that fixes the rod inserted into the rod orifice is screwed, and a screw member orifice (13) into which a screw member (5) to be screwed into the vertebra is inserted, and that connects the rod and the screw member to be screwed into the vertebra; and a diameter adjusting member (4) that is inserted into the rod orifice and has formed therein a convex curved surface (21) that abuts against the rod orifice and a concave curved surface (22) that abuts against the rod. The screw member is inserted into the screw member orifice and fixed to the connector, and the rod is inserted into the rod orifice into which the diameter adjusting member has been inserted and is fixed to the connector.

## Description

### BACKGROUND OF THE INVENTION

### Field of the Invention

The present invention relates to a connector for a vertebra connection member that connects a plurality of vertebrae via a rod.

### Description of the Related Art

Examples of known vertebra connection members that connect a plurality of vertebrae include a connection member described in a pamphlet "The ISOLA Spinal System" DS002P01 200503 published by ROBERT REID INC. and a connection member described in a pamphlet "Monarch SPINE SYSTEM" DS026P01 200505 published by ROBERT REID INC.

Such connectors connect screw members that are screwed into a plurality of vertebrae and the rod in separate positions. The connector is formed by a rod insertion portion having a rod orifice into which the rod is inserted and a screw member insertion portion that extends from the rod insertion portion sidewise of the rod and has a screw member orifice into which the screw member is inserted. The screw members and rod are connected in separate positions by inserting the screw members screwed into a plurality of vertebrae into the screw member insertion portions, fixing the screw members, inserting the rod into the rod insertion portions, and fixing the rod.

### SUMMARY OF THE INVENTION

However, since the vertebra connection members are applied to a variety of people, for example, where there is a difference in the structure of body or muscular power, as between children and adults, the diameter of rod used in the vertebra connection members has to be changed.

However, the problem associated with the conventional connectors is that because connectors of a large number of types corresponding to the diameter of each rod have to be prepared, the cost is high.

Accordingly, it is an object of the present invention to provide a connector that can be adapted to rods of a plurality of diameters and can reduce cost.

The connector in accordance with the present invention is a connector for vertebra connection members that connect a plurality of vertebrae via a rod, comprising: a connector body that has formed therein a rod orifice into which the rod is inserted, a fixing member orifice into which a fixing member that fixes the rod inserted into the rod orifice is screwed, and a screw member orifice into which a screw member to be screwed into the vertebra is inserted, and that connects the rod and the screw member to be screwed into the vertebra; and a diameter adjusting member that is inserted into the rod orifice and has formed therein a convex curved surface that abuts against the rod orifice and a concave curved surface that abuts against the rod.

In the connector in accordance with the present invention, where the diameter adjusting member is inserted into the rod orifice of the connector body, the convex curved surface of the diameter adjusting member abuts against the rod orifice. Further, where the rod is inserted into the rod orifice, the concave curved surface of the diameter adjusting member abuts against the rod. Where the fixing member is screwed into the fixing member orifice of the connector body, the rod and the diameter adjusting member are pushed into the rod orifice, whereby the rod is fixed to the connector. Because the rod is thus fixed to the connector via the diameter adjusting member, rods of a plurality of diameters can be fixed to the connector body of the same shape by inserting the diameter adjusting member having formed therein the concave curved surface corresponding to the rod diameter into the rod orifice. Because the connector body can thus be adapted to rods of a plurality of diameters, the connector body can be imparted with general versatility and, therefore, the connector cost can be reduced.

It is preferred that the convex curved surface be formed to conform to a shape of part of the rod orifice, and the concave curved surface be formed to conform to a shape of part of the rod. In such connector, because the diameter adjusting member is formed so as to conform to the shape of part of the rod orifice and the rod, the rod can be fixed to the connector body, without a play, when the fixing member is screwed into the connector body.

It is preferred that the convex curved surface be formed to conform to a shape of part of the rod orifice, and the concave curved surface be formed into a shape that abuts against the rod in two or more places. It is also preferred that the convex curved surface be formed into a shape that abuts against the rod orifice in two or more places, and the concave curved surface be formed to conform to a shape of part of the rod. It is also preferred that the convex curved surface be formed into a shape that abuts against the rod orifice in two or more places, and the concave curved surface be formed into a shape that abuts against the rod in two or more places. With these connectors, where the fixing member is screwed into the fixing member orifice, the rod is directly or indirectly supported in three or more places by the fixing member and the rod orifice. As a result, the rod can be fixed to the connector body, without a play.

The rod orifice is preferably formed into an elliptical shape with a long diameter direction along a screw-in direction of the fixing member. With such a connector, because the diameter of the rod orifice in the direction in which the fixing member is screwed into is increased, an extra space is provided in the rod orifice when the diameter adjusting member and the rod are inserted. As a result, the diameter adjusting member and the rod, or the diameter adjusting member or the rod can be easily inserted into the rod orifice.

Further, it is preferred that the diameter adjusting member have formed therein a protrusion portion extending outwardly from the convex curved surface, and that the connector body have formed therein a depression portion to which the protrusion portion conforms when the diameter adjusting member is inserted into the rod orifice. With such connector, the protrusion portion formed in the diameter adjusting member is mated with the depression portion formed in the rod orifice. Therefore, the diameter adjusting member inserted into the rod orifice can be easily positioned.

Further, it is preferred that the depression portion be formed at both ends in a longitudinal direction of the rod orifice, and the protrusion portion be formed in a position corresponding to the depression portion when the diameter adjusting member is inserted into the rod orifice. With such a connector, when the diameter adjusting member is inserted into the rod orifice, the protrusion portion of the diameter adjusting member is mated with the concave curved surface so that the protrusion portion pinch the rod orifice. As a result, the diameter adjusting member can be prevented from falling out from the rod orifice.

It is also preferred that the diameter adjusting member be formed to have a length substantially equal to a length of the rod orifice. With such a connector, the diameter adjusting member inserted into the rod orifice does not protrude outwardly from the rod orifice and protrusions and depressions of the connector contour can be reduced to a minimum. As a result, a load applied to the peripheral tissue of the vertebra can be reduced.

The present invention can be more thoroughly understood based on the detailed explanation and appended drawings presented below. These drawings are presented merely to illustrate the invention and should not be construed as placing any limitation thereon.

### BRIEF DESCRIPTION OF THE DRAWINGS

FIG. 1 is a perspective view illustrating the state in which three vertebra connection members using the connector of the embodiment of the present invention are connected;
FIG. 2 is a front view of the vertebra connection member;
FIG. 3 is a front view of the screw member;
In FIG. 4, (a) is a front view of the connector, (b) is a top view of the connector; (c) is a front perspective view of the connector; and (d) is a rear perspective view of the connector;
In FIG. 5, (a) is a perspective view of the fixing member; (b) is a front view of the fixing member; and (c) is a top view of the fixing member;
In FIG. 6, (a) is a perspective view of the diameter adjusting member; and (b) is a top view of the diameter adjusting member;
FIG. 7 is a front view illustrating a state in which the screw member is bent;
In FIG. 8, (a) is a front view of the vertebra connection member using the diameter adjusting member with a different shape of the convex curved surface; (b) is a front view of the vertebra connection member using the diameter adjusting member with a different shape of the concave curved surface; and (c) is a front view of the vertebra connection member using the diameter adjusting member with a different shape of the convex curved surface and concave curved surface;
In FIG. 9, (a) is a side view of the diameter adjusting member with a different shape of the protrusion portion; (b) is a front perspective view of the diameter adjusting member with a different shape of the protrusion portion; and (c) is a rear perspective view of the diameter adjusting member with a different shape of the protrusion portion;
In FIG. 10, (a) is a front view of the connector body with a different shape of the depression portion; (b) is a front perspective view of the connector body with a different shape of the depression portion; and (c) is a rear perspective view of the connector body with a different shape of the depression portion;
In FIG. 11, (a) is a side view of the diameter adjusting member in which no protrusion portion has been formed; (b) is a front perspective view of the diameter adjusting member in which no protrusion portion has been formed; and (c) is a rear perspective view of the diameter adjusting member in which no protrusion portion has been formed; and
In FIG. 12, (a) is a front view of the connector body in which no depression portion has been formed; (b) is a front perspective view of the connector body in which no depression portion has been formed; and (c) is a rear perspective view of the connector body in which no depression portion has been formed.

### DESCRIPTION OF THE PREFERRED EMBODIMENTS

Embodiments of the present invention will be described below in greater detail with reference to the drawings. In the explanation of the drawings, identical elements will be assigned with identical reference symbols and redundant explanation thereof will be omitted.

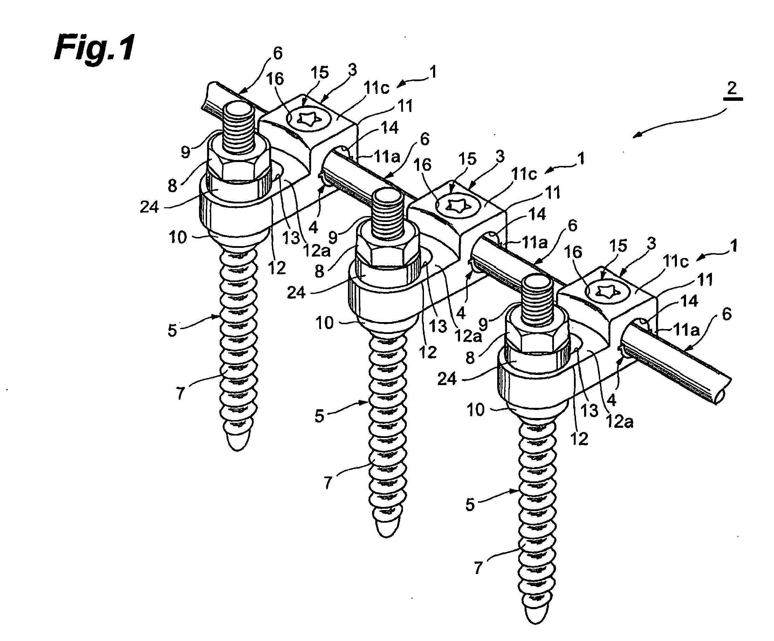

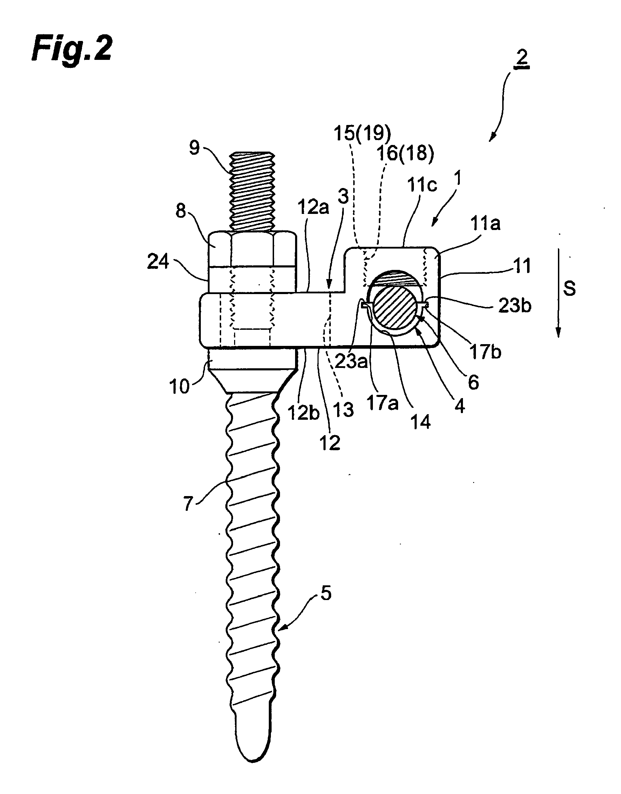

FIG. 1 is a perspective view illustrating the state in which three vertebra connection members using the connector of the embodiment of the present invention are connected. FIG. 2 is a front view of the vertebra connection member.

As shown in FIG. 1 and FIG. 2, a connector 1 of the present embodiment connects a screw member 5 to be screwed into a vertebra and a rod 6 in a vertebra connection member 2 that connects and fixes a plurality of vertebrae. The connector 1 comprises a connector body 3 and a diameter adjusting member 4.

The rod 6 is formed into a round columnar shape and a length such that a plurality of vertebra connection members 2 can be connected. The rod 6 is formed from a corrosion-resistant material such as titanium, and a coating film is formed on the entire surface thereof by anodization.

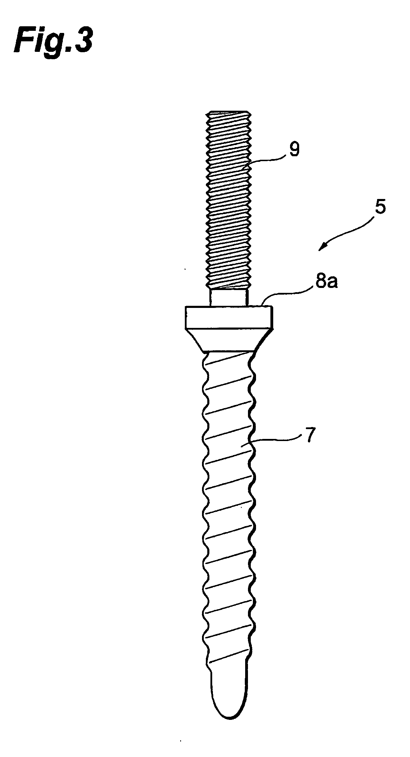

The screw member 5 is screwed into a vertebra. As shown in FIG. 1, FIG. 2, and FIG. 3, the screw member 5 is formed into a rod-like shape, and a first male threaded portion 7 to be screwed into the vertebra is formed at one end of the screw member 5. A second male threaded portion 9 onto which a nut 8 is screwed is formed at the other end of the screw member 5. A locking portion 10 that is larger in diameter than other portions is formed between the first male threaded portion 7 and the second male threaded portion 9. An upper end surface 8a (upper end surface in FIG. 3) of the locking portion 10 is formed into a flat shape. The screw member 5 is formed from a corrosion-resistant material such as titanium, and a coating film is formed on the entire surface thereof by anodization.

As shown in FIG. 1, the connector body 3 serves to connect the screw member 5 and the rod 6. As shown in FIG. 1, FIG. 2, and FIG. 4, the connector body 3 comprises a rod fixing portion 11 for fixing the rod 6 and a screw member fixing portion 12 for fixing the screw member 5. The connector body 3 is formed from a corrosion-resistant material such as titanium, and a coating film is formed on the entire surface thereof by anodization.

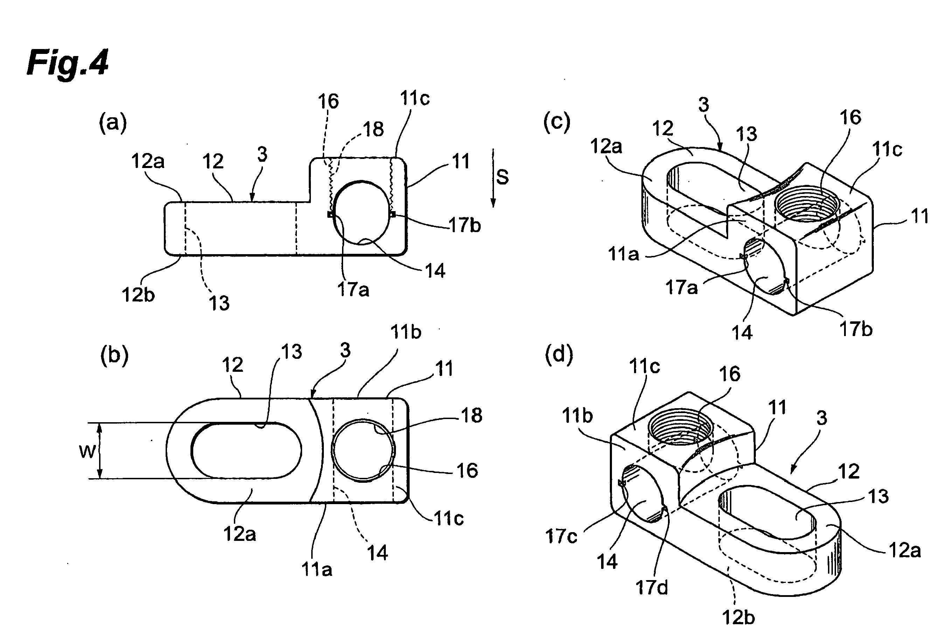

The screw member fixing portion 12 is long and narrow and extends sidewise from the lower portion of the side surface of the rod fixing portion 11. A screw member orifice 13 into which the second male threaded portion 9 of the screw member 5 is inserted is formed in the screw member fixing portion 12 so as to pass through the screw member fixing portion 12 from an upper surface 12a (upper surface in (a) of FIG. 4) of the screw member insertion portion to a lower surface 12b (lower surface in (a) of FIG. 4) of the screw member fixing portion. The screw member orifice 13 is formed to be elongated along the rod fixing portion 11, so that the distance from the inserted screw member 5 to the rod fixing portion 11 can be adjusted. The width W (see (d) of FIG. 4) of the screw member orifice 13 is formed to be larger than the diameter of the second male threaded portion 9 and smaller than the diameter of the locking portion 10.

In the rod fixing portion 11, a rod orifice 14 into which the rod 6 and diameter adjusting member 4 are inserted is formed through the rod fixing portion 11 from a front surface 11a (lower surface in (b) of FIG. 4) to a rear surface (upper surface in (b) of FIG. 4) of the rod fixing portion, and a fixing member orifice 16 into which the fixing member 15 that fixes the rod 6 inserted into the rod orifice 14 is screwed is formed through the rod fixing member 11 from an upper surface 11c (upper surface in (a) of FIG. 4) of the rod fixing potion to the rod orifice 14, so as to be perpendicular to the rod orifice 14. The direction in which the fixing member 15 is screwed into the fixing member orifice 16 is called a screw-in direction S (arrow S in FIG. 2 and (a) of FIG. 4).

The rod orifice 14 is formed into an elliptical shape such that the long diameter direction thereof is along the screw-in direction S of the fixing member 15. Further, the diameter in the direction perpendicular to the screw-in direction S is equal to the maximum diameter of the rod 6 that is wished to be used, or larger than the maximum diameter of the rod 6 that is wished to be used.

In the front surface 11a of the rod fixing portion of the rod fixing member 11, depression portions 17a, 17b that recede from the rod orifice 14 in the direction perpendicular to the screw-in direction S and from the front surface 11 a of the rod fixing portion to the rear surface 11b of the rod fixing portion are formed on both sides of the central portion of the rod orifice 14 in the screw-in direction S. Further, in the rear surface 11b of the rod fixing portion of the rod fixing member 11, depression portions 17c, 17d that recede from the rod orifice 14 in the direction perpendicular to the screw-in direction S and from the rear surface 11b of the rod fixing portion to the front surface 11a of the rod fixing portion are formed on both sides of the central portion of the rod orifice 14 in the screw-in direction S.

A female threaded portion 18 into which the fixing member 15 is screwed is formed in the fixing member orifice 16. The diameter of the fixing member orifice 16 is larger than the diameter of the rod orifice 14. In the present embodiment, the diameter of the fixing member orifices is made larger than the diameter of the rod orifice 14, but it may be equal to the diameter of the rod orifice 14 or may be smaller than the diameter of the rod orifice 14.

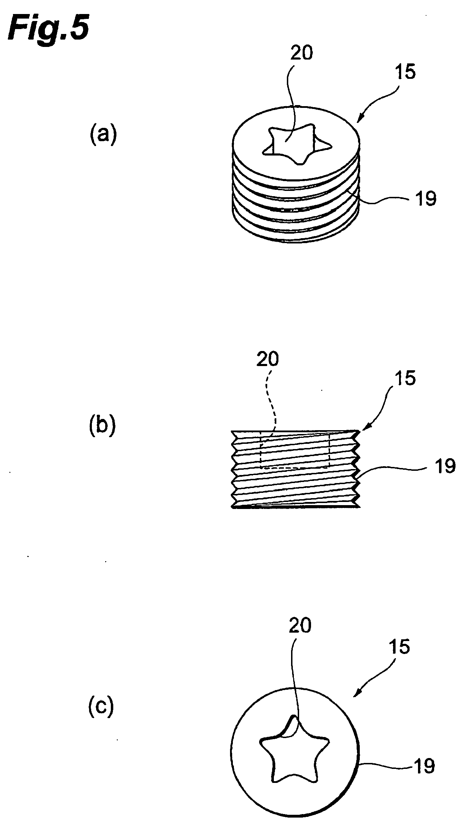

As shown in FIG. 1 and FIG. 2, the fixing member 15 is screwed into the fixing member orifice 16 of the connector body 3 and serves to fix the rod 6 to the connector 1. As shown in FIG. 5, the fixing member 15 is formed into a round columnar shape. A male threaded portion 19 that conforms to the female threaded portion 18 of the rod orifice 14 is formed at the side surface of the fixing member 15. The upper and lower surfaces (upper and lower surfaces in (b) of FIG. 5) of the fixing member 15 are formed into a flat shape, and a star-shaped depression portion 20 into which a TORX (star-shaped) wrench is inserted is formed in an upper surface of the fixing member 15. The fixing member 15 is formed from a corrosion-resistant material such as titanium, and a coating film is formed on the entire surface thereof by anodization.

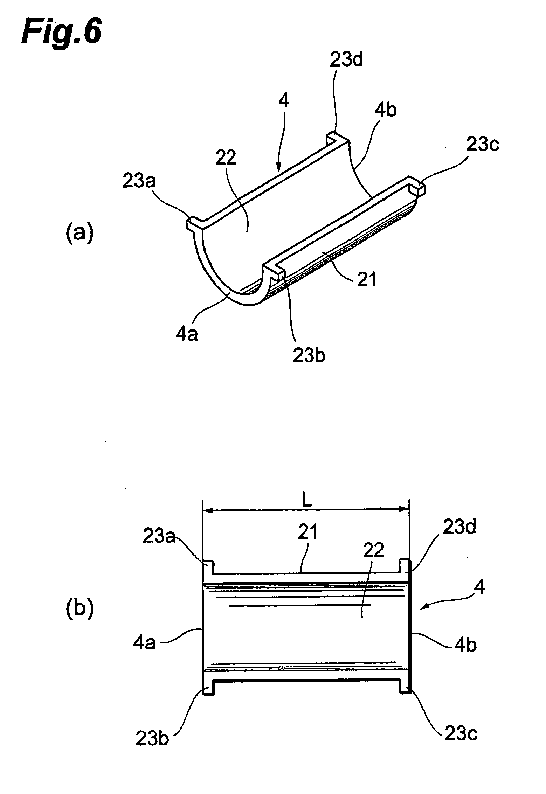

As shown in FIG. 1 and FIG. 2, the diameter adjusting member 4 is inserted between the rod 6 and rod orifice 14 and serves to adjust the shape (or diameter) of the rod 6 in relation to the shape (or diameter) of the rod orifice 14. As shown in FIG. 6, the diameter adjusting member 4 is formed into a half-pipe shape. The length L (see (b) of FIG. 6) of the diameter adjusting member 4 is substantially equal to the length of the rod orifice 14. A convex curved surface 21 that conforms to the shape of the front half (in the screw-in direction S) of the inner wall surface of the rod orifice 14 is formed at the outer peripheral surface of the diameter adjusting member 4. A concave curved surface 22 that conforms to the shape of the semicylindrical portion of the outer peripheral surface of the rod 6 is formed at the inner peripheral surface of the diameter adjusting member 4.

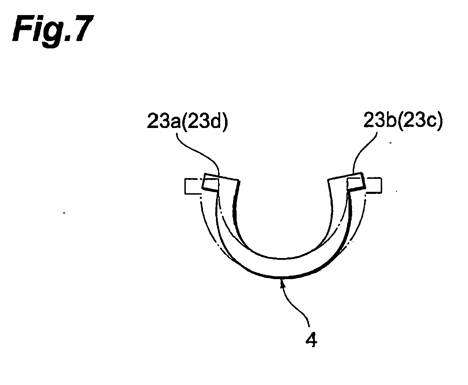

Further, in the diameter adjusting member 4, protrusion portions 23a to 23d extending outwardly from the respective convex curved surfaces 21 are formed at both ends in the circular arc direction of a front surface side 4a (left side in (b) of FIG. 6) of the diameter adjusting member and a rear surface side 4b (right side in (b) of FIG. 6) in the positions corresponding to the depression portions 17a to 17d (see FIG. 4) of the connector body 3 when the diameter adjusting member 4 is inserted in the rod orifice 14. The protrusion portions 23a to 23d are formed into a shape conforming to the shape of the depression portions 17a to 17d of the rod fixing portion 11 when the diameter adjusting member 4 is inserted in the rod orifice 14. The diameter adjusting member 4 is formed from a corrosion-resistant material such as titanium, and a coating film is formed on the entire surface thereof by anodization. Further, the diameter adjusting member 4 can have predetermined elastic properties and, as shown in FIG. 7, the protruding portions 23a to 23d can be bent toward the center of the diameter adjusting member 4 by elastic deformation.

A method for connecting the screw member 5 screwed into the vertebra and the rod 6 by using the connector 1 of the present embodiment will be described below.

First, a diameter adjusting member 4 is prepared that has formed therein a concave curved surface 22 conforming to the diameter of the rod 6 to be used. Then, as shown in FIG. 7, the diameter adjusting member 4 is elastically deformed so that the protrusion portions 23a to 23d deform elastically toward the center of the diameter adjusting member 4, and the diameter adjusting member is inserted into the rod orifice 14 of the connector body 3. Where the diameter adjusting member 4 is completely inserted into the rod orifice 14, the diameter adjusting member 4 restores the original state, and the protrusion portions 23a to 23d of the diameter adjusting member 4 are mated with respective depression portions 17a to 17d of the connector body 3. As a result, the connector body 3 and diameter adjusting member 4 are integrated and the connector 1 is assembled.

Then, the second male threaded portion 9 of the screw member 5 screwed into the vertebra is inserted into the screw member orifice 13, and the lower surface 12b of the screw member fixing portion is abutted against the locking portion 10. The position of the connector 1 with respect to the screw member 5 is then adjusted so that the direction of the rod orifice 14 of the connector 1 coincides with the direction of the rod orifice 14 of the connector 1 that will fix that screw member 5 screwed into the other vertebra. Where the position of the connector body 3 in relation to the screw member 5 is determined, the screw member 5 is held in this position and the nut 8 is screwed onto the second male threaded portion via a washer 24. Then, the screw member fixing portion 12 is squeezed between the locking portion 10 and nut 8, and the connector 1 is fixed to the screw member 5.

Then, the rod 6 is inserted into the rod orifice 14 and abutted against the concave curved surface 22 of the diameter adjusting member 4. The rod 6 is then moved and the position of the rod 6 with respect to the connector 1 is adjusted. Where the position of the rod 6 with respect to the connector 1 is determined, the rod 6 is held in this position and the fixing member 15 is screwed into the fixing member orifice 16. Where the fixing member 15 is screwed into the fixing member orifice 16, the fixing member 15 abuts against the rod 6 inside the rod orifice 14, and where the fixing member 15 is further screwed in, the fixing member 15 pushes the rod 6 in the screw-in direction S. At this time, the rod 6 is pressed against the concave curved surface 22 of the diameter adjusting member 4, and the diameter adjusting member 4 is pressed against the rod orifice 14. As a result, the rod 6 is fixed to the connector 1 by pressing against the rod orifice 14 via the diameter adjusting member 4.

The screw member 5 screwed into the vertebra and the rod 6 are thus connected by the connector 1.

As described hereinabove, in the connector 1 of the present embodiment, the diameter adjusting member 4 comprising a concave curved surface 22 conforming to the shape of part of the outer peripheral surface of the rod 6 and the convex curved surface 21 conforming to the shape of part of the inner wall surface of the rod orifice 14 is inserted into the rod orifice 14 of the connector body 3, and the rod 6 is inserted into the rod orifice 14 into which the diameter adjusting member 4 has been inserted. Therefore, where a plurality of types of diameter adjusting member 4 that are easier to process than the connector body 3 are prepared correspondingly to rods 6 of different diameters, the rods 6 of different diameters can be fixed with the connector body 3 of the same shape. Thus, the connector body 3 can be adapted to rods 6 of a plurality of diameters and imparted with general utility and, therefore, the connector cost can be reduced.

Further, with the connector 1 of the present embodiment, because the convex curved surface 21 is formed to conform to the shape of part of the rod orifice 14, and the concave curved surface 22 is formed to conform to the shape of part of the rod 6, the rod 6 can be fixed to the connector 1, without a play, when the fixing member 15 is inserted into the connector body.

Further, with the connector 1 of the present embodiment, because the rod orifice 14 is formed into an elliptical shape with a large diameter in the screw-in direction S, extra space is provided when the diameter adjusting member 4 and rod 6 are inserted. Therefore, the diameter adjusting member 4 and rod 6 can be easily inserted into the rod orifice 14.

Further, with the connector 1 of the present embodiment, because the protrusion portions 23a to 23d formed in the diameter adjusting member 4 are mated with the depression portions 17a to 17d formed in the rod orifice 14, positioning of the diameter adjusting member 4 inserted into the rod orifice 14 is facilitated.

Further, with the connector 1 of the present embodiment, where the diameter adjusting member 4 is inserted into the rod orifice 14, the protrusion portions 23a to 23d of the diameter adjusting member 4 are mated with the depression portions 17a to 17d so that the protrusion portion 23a to 23d pinch the rod orifice 14. As a result, the diameter adjusting member 4 can be prevented from falling out from the rod orifice 14.

Further, with the connector 1 of the present embodiment, the diameter adjusting member 4 is formed to have a length substantially equal to the length of the rod orifice 14. Therefore, the diameter adjusting member 4 inserted into the rod orifice 14 does not protrude at all or almost at all from the rod orifice 14 and protrusions and depressions of the contour of connector 1 can be reduced to a minimum. As a result, a load applied to the peripheral tissue of the vertebrae connected by the vertebra connection member 2 using the connector 1 can be reduced.

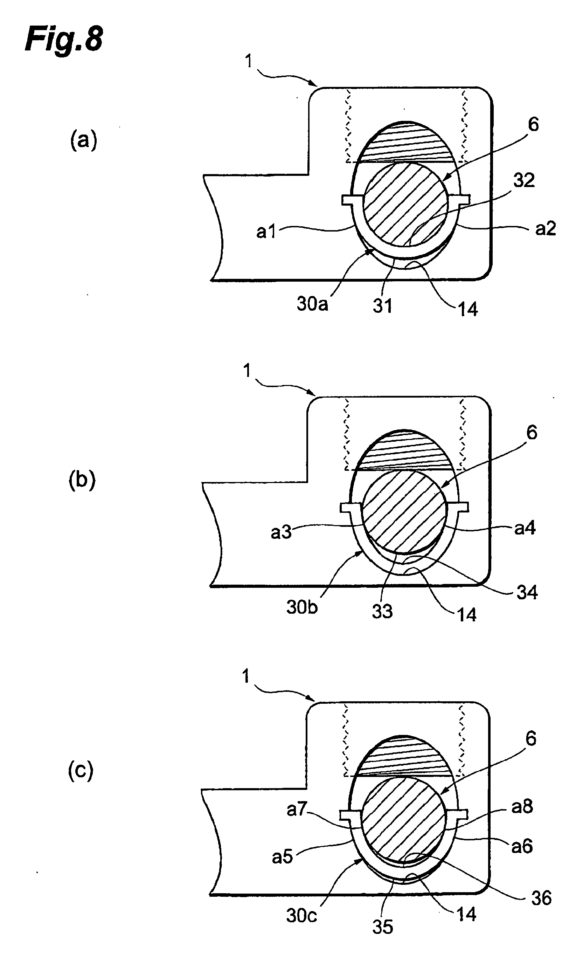

The present invention has been specifically explained hereinabove based on the embodiment thereof, but the present invention is not limited to the above-described embodiment. For example, in the above-descried embodiment, the configuration is explained in which the convex curved surface 21 of the diameter adjusting member 4 is formed to conform with the shape of part of the rod orifice 14, and the concave curved surface 22 of the diameter adjusting member 4 is formed to conform with the shape of part of the rod 6, but such shape is not limiting. For example, a convex curved surface 31 may be formed into a shape that abuts against the rod 6 is two locations (or two or more locations) of contact points a1, a2, and a concave curved surface 32 may be formed to conform to the shape of part of the rod 6, as in a diameter adjusting member 30a shown in (a) of FIG. 8. Further, a convex curved surface 33 may be formed to conform to the shape of part of the rod orifice 14, and a concave curved surface 34 may be formed into a shape that abuts against the rod 6 is two locations (or two or more locations) of contact points a3, a4, as in a diameter adjusting member 30b shown in (b) of FIG. 8. Alternatively, a convex curved surface 35 may be formed into a shape that abuts against the rod orifice 14 is two locations (or two or more locations) of contact points a5, a6, and a concave curved surface 36 may be formed into a shape that abuts against the rod 6 is two locations (or two or more locations) of contact points a7, a8, as in a diameter adjusting member 30c shown in (c) of FIG. 8.

As a result, when the fixing member 15 is screwed into the fixing member orifice 16, the rod 6 is directly or indirectly supported in three or more places by the fixing member 15 and rod orifice 14 via the diameter adjusting member 4. As a result, the rod can be fixed to the connector body, without a play. In this case, the contact point a1 and contact point a2, the contact point a3 and contact point a4, the contact point a5 and contact point a6, and the contact point a7 and contact point a8 are preferably positioned with a left-right symmetry with respect to the line in the screw-in direction S that passes through the contact points of the rod 6 and fixing member 15.

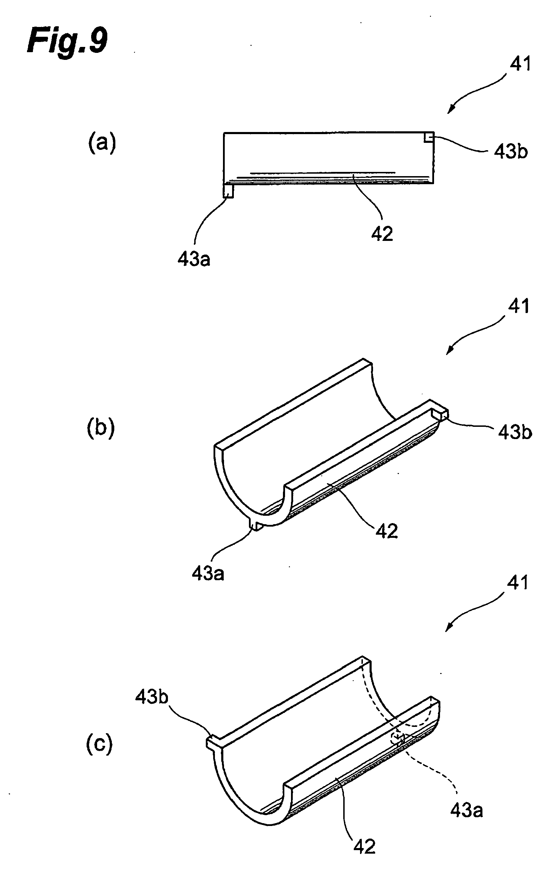

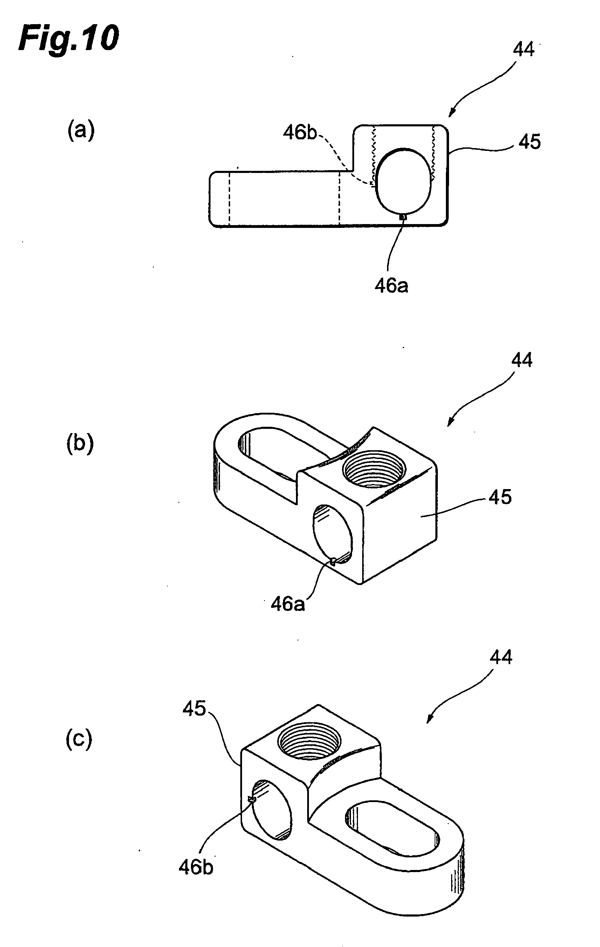

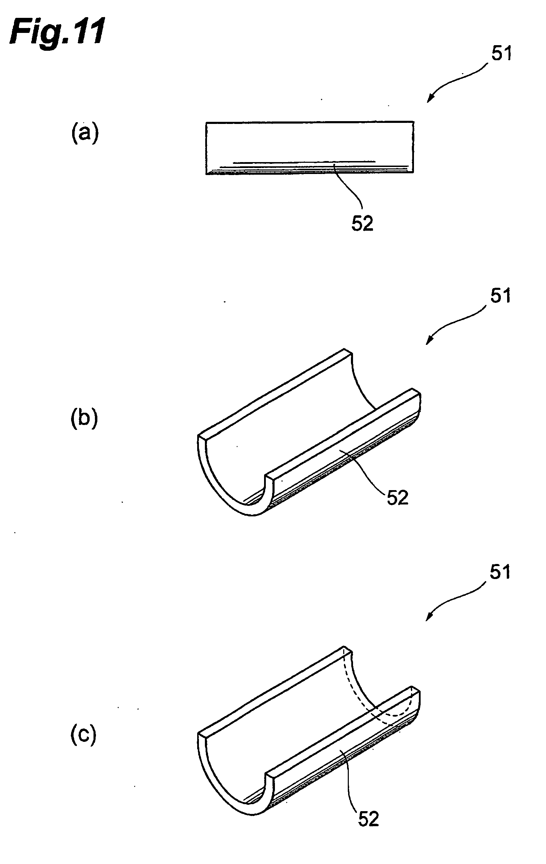

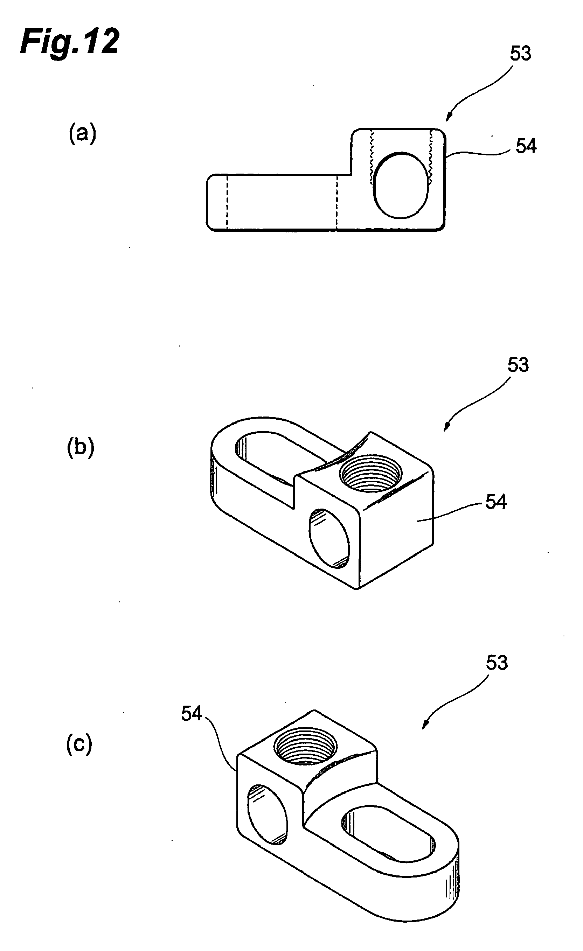

In the above-described embodiment, a configuration is explained in which the depression portions 17a to 17d formed in the connector body 3 are formed on both sides of the central portion in the screw-in direction S in the rod orifice 14, and the protrusion portions 23a to 23d formed in the diameter adjusting member are formed in the positions corresponding to the depression portions 17a to 17d, but the positions in which the depression portions or the protrusion portions are formed and the number thereof are not particularly limited, and either the depression portions or the protrusion portions, or both the depression portions and the protrusion portions may not be formed. For example, a protrusion portion 43a extending outwardly from a lower portion (portion on the lower side in (a) of FIG. 9) of the convex curved surface 42 may be formed at one end, a protrusion portion 43b extending outwardly from a side portion (portion on the front side in (a) of FIG. 9) of the convex curved surface 42 may be formed on the other end, as in a diameter adjusting member 41 shown in FIG. 9, and depression portions 46a, 46b may be formed in the positions corresponding to the protrusion portions 43a, 43b of the diameter adjusting member 41 in a rod fixing portion 45, as in the connector body 44 shown in FIG. 10. On the other hand, for example, a protrusion portion may not be formed in a convex curved surface 52 as in a diameter adjusting member 51 shown in FIG. 11, and also a depression portion may not be formed in the rod fixing portion 54 as in the connector body 53 shown in FIG. 12.

The explanation of the present invention presented hereinabove clearly demonstrates that various modifications of the present invention can be made. Such modifications should not deviate from the spirit and scope of the present invention, and all the improvements obvious to a person skilled in the art are included in the claims below.

## Claims

1. A connector for vertebra connection members that connect a plurality of vertebrae via a rod, comprising:
a connector body that has formed therein a rod orifice into which the rod is inserted, a fixing member orifice into which a fixing member that fixes the rod inserted into the rod orifice is screwed, and a screw member orifice into which a screw member to be screwed into the vertebra is inserted, and that connects the rod and the screw member to be screwed into the vertebra; and
a diameter adjusting member that is inserted into the rod orifice and has formed therein a convex curved surface that abuts against the rod orifice and a concave curved surface that abuts against the rod.

2. The connector according to claim 1, wherein
the convex curved surface is formed to conform to a shape of part of the rod orifice; and
the concave curved surface is formed to conform to a shape of part of the rod.

3. The connector according to claim 1, wherein
the convex curved surface is formed to conform to a shape of part of the rod orifice; and
the concave curved surface is formed into a shape that abuts against the rod in two or more places.

4. The connector according to claim 1, wherein
the convex curved surface is formed into a shape that abuts against the rod orifice in two or more places; and
the concave curved surface is formed to conform to a shape of part of the rod.

5. The connector according to claim 1, wherein
the convex curved surface is formed into a shape that abuts against the rod orifice in two or more places; and
the concave curved surface is formed into a shape that abuts against the rod in two or more places.

6. The connector according to claim 1, wherein
the rod orifice is formed into an elliptical shape with a long diameter direction along a screw-in direction of the fixing member.

7. The connector according to claim 1, wherein
the diameter adjusting member has formed therein a protrusion portion extending outwardly from the convex curved surface; and
the connector body has formed therein a depression portion to which the protrusion portion conforms when the diameter adjusting member is inserted into the rod orifice.

8. The connector according to claim 1, wherein
the depression portion is formed at both ends in a longitudinal direction of the rod orifice; and
the protrusion portion is formed in a position corresponding to the depression portion when the diameter adjusting member is inserted into the rod orifice.

9. The connector according to claim 7, wherein
the diameter adjusting member is formed to have a length substantially equal to a length of the rod orifice.
